(19)

Europäisches
Patentamt
European
Patent Office
Office européen
des brevets

(11) **EP 4 467 081 A1**

(12) **EUROPEAN PATENT APPLICATION**

(43) Date of publication:
**27.11.2024 Bulletin 2024/48**

(21) Application number: **23174686.8**

(22) Date of filing: **22.05.2023**

(51) International Patent Classification (IPC):
**A61B 8/00** *(2006.01)*        **A61B 8/08** *(2006.01)*

(52) Cooperative Patent Classification (CPC):
**A61B 8/4477; A61B 8/481; A61B 8/5223;**
A61B 8/08; A61B 8/085; A61B 8/0883; A61B 8/0891

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universität Bern**
**3012 Bern (CH)**

(72) Inventors:
• **Hahne, Christopher**
  **3006 Bern (CH)**
• **Sznitman, Raphael**
  **1006 Lausanne (CH)**

(74) Representative: **Vesterinen, Jussi Tapio**
**LUMI IP GmbH**
**Rodtmattstrasse 45**
**3014 Bern (CH)**

(54) **GEOMETRIC LOCALISATION IN MEDICAL IMAGING**

(57)    A geometric ultrasound localisation microscopy method is provided according to one embodiment for localising microbubbles. The proposed method uses a novel geometry framework for microbubble localisation through ellipse or ellipsoid intersections to overcome limitations inherent to beamforming. This approach provides a finer distinction between overlapping and clustered spots, improving localisation precision, reliability, and computation efficiency. The present invention thus challenges the conventional wisdom that beamforming is necessary for ULM and proposes a novel beamformer-free approach that according to one example relies on time difference of arrival information between echoes for microbubble localisation.

Fig. 4

Flowchart:
- Carry out data acquisition from arbitrarily positioned sensors. — 101
- Pre-process the acquired data. — 102
- Detect echoes from the pre-processed data. — 103
- Extract features from the detected echoes. — 104
- Carry out an echo feature matching process based on the extracted features. — 105
- Construct ellipses for the current sensor and for the best matching echoes received by the other sensors. — 106
- Determine the intersection point of the ellipses to localise a respective microbubble. — 107
- Fuse localisation candidates to improve precision. — 108
- End

EP 4 467 081 A1

**Description**

FIELD OF THE INVENTION

The present invention relates to a method for performing geometric localisation in medical imaging. One example embodiment of the invention concerns geometric ultrasound localisation microscopy. More specifically, the proposed method uses an echo correspondence determination process instead of beamforming as traditionally done in ultrasound localisation microscopy. The present invention also relates to a sensor system for implementing the steps of the method.

BACKGROUND OF THE INVENTION

Contrast-enhanced ultrasound (CEUS) has become a viable method for non-invasive, dynamic visualisation of a human cardiovascular system for instance, yet ultrasound localisation microscopy (ULM), which can be considered as the successor for CEUS, has enabled a revolutionary breakthrough by offering ten times higher resolution. To date, delay-and-sum (DAS) beamformers are used to render ULM frames, ultimately determining the image resolution capability.

ULM is a non-invasive imaging technique deep into organs at microscopic scales. It works by using microscopic bubbles, referred to also as microbubbles, circulated through the bloodstream as a contrast agent to measure the reflection of high-frequency acoustic waves passing through the body. ULM has revolutionised medical imaging by enabling sub-wavelength resolution from images acquired by piezo-electric transducers and computational beamforming. However, the necessity of beamforming for ULM remains questionable. The introduction of ULM has recently surpassed the diffraction-limited spatial resolution and enabled highly detailed visualisation of the vascular system. ULM borrows concepts from super-resolution fluorescence microscopy techniques to precisely locate individual particles with sub-pixel accuracy over multiple frames. By the accumulation of all localisations over time, ULM can produce a super-resolved image, providing researchers and clinicians with highly detailed representation of the vascular structure and function.

While CEUS is used in the identification of musculoskeletal soft tissue tumours, the far higher resolution capability offered by ULM has great potential for clinical translation to improve the reliability of cancer diagnosis (i.e., enable differentiation of tumour types in kidney cancer or detect breast cancer tissue). Moreover, ULM has shown promise in imaging neurovascular activity after visual stimulation (functional ULM). Pioneering studies on ULM image reconstruction include the ground-breaking work by Errico et al. "Ultrafast ultrasound localization microscopy for deep super-resolution vascular imaging", Nature 527(7579), 499-502 (2015), which demonstrated the potential of ULM to successfully localise contrast agent particles (microbubbles) using a two-dimensional (2D) point-spread-function model. In general, the accuracy in microbubble localisation is key to achieving sub-wavelength resolution.

However, the conventional approach for ULM involves using computational beamformers, which may not be ideal for microbubble localisation. Beamforming techniques have been developed to handle irregular topologies, whereas microbubbles exhibit a uniform geometric structure, for which ULM only requires information about its spatial position. Although the impact of adaptive beamforming has been studied for ULM to investigate its potential to refine microbubble localisation, optimisation of the point-spread function (PSF) poses high demands on the transducer array, data storage, and algorithm complexity.

It is to be noted that the above-identified issues or similar issues may also be present in other medical imaging localisation methods. These methods may be used to track microbubbles and/or particles in a human or animal body.

BRIEF DESCRIPTION OF THE INVENTION

The present invention aims to overcome at least some of the above-identified problems. More specifically, to overcome the above problems, the present invention proposes a new medical imaging method for localising microbubbles and/or particles in a human or animal body by using a sensor system, where the method is based on an echo correspondence determination process. One example embodiment relates to an alternative approach for ULM that does not use any beamformers and thus omits beamforming from the processing pipeline for the first time. The present invention, as explained later, challenges the conventional assumption that beamforming is the ideal pre-processing step for ULM and presents an alternative approach based on geometric reconstruction from time difference of arrival (TDOA) information for instance.

According to a first aspect of the invention, a medical imaging method for localising microbubbles and/or particles is proposed as recited in claim 1.

The proposed method uses a novel geometry framework for microbubble and/or particle localisation through ellipse or ellipsoid intersections to overcome limitations inherent to beamforming. This approach provides a finer distinction between overlapping and clustered spots, improving localisation precision, reliability, and computation efficiency. The present invention thus challenges the conventional wisdom that beamforming is necessary (e.g., for ULM) and proposes a novel beamformer-free approach that according to one example relies on TDOA information for microbubble and/or particle localisation. The proposed approach demonstrates promising results and leads to high precision, requires only very limited computational resources and needs only little memory.

According to a second aspect of the invention, a sensor system is provided, which is configured to implement the method according to the first aspect of the present invention.

Other aspects of the invention are recited in the dependent claims attached hereto.

BRIEF DESCRIPTION OF THE DRAWINGS

The invention will now be described in more detail with reference to the attached drawings, in which:

- Figure 1 illustrates a geometric model of two ellipses showing one virtual source and two receivers;

- Figure 2 illustrates in a simplified manner the proposed method and highlights the differences to a state-of the art ULM method;

- Figure 3 shows an echo feature extraction and echo correspondence architecture to extract echo features and to determine mutually matching echoes across a plurality of sensor channels;

- Figure 4 shows a flowchart illustrating the proposed ULM microbubble localisation method according to an example embodiment of the present invention; and

- Figure 5 illustrates visually the echo correspondence determination used in the proposed ULM microbubble localisation method according to an example of the present invention.

DETAILED DESCRIPTION OF THE INVENTION

It should be noted that the figures are provided merely as an aid to understanding the principles underlying the invention, and should not be taken as limiting the scope of protection sought. Where the same reference numbers are used in different figures, these are intended to indicate similar or corresponding features. As used herein, unless otherwise specified the use of the ordinal adjectives "first", "second", "third", etc. to describe a common object, merely indicate that different instances of like or different objects are being referred to, and are not intended to imply that the objects so described must be in a given sequence, either temporally, spatially, in ranking, or in any other manner. Bold fonts are used to describe vectors (lower-case letter) or matrices (upper-case letter), where the vectors are to be understood as a finite sequence of numbers of a fixed length.

Some definitions that help understand the teachings of the present invention are given next.

A sensor, detector or transducer channel or sensor continuously records the amplitude of energy packets that physically arrive on the sensing surface at different points in time. A sensor is generally only responsive to a certain light, electromagnetic or sonic wave spectrum. In the embodiment described below, sensors are ultrasound transducers.

A data frame, or simply frame, is characterised by a set of sensor amplitudes recorded consecutively at a certain time interval. In time-of-flight applications, this time interval typically starts with the emittance of a pulse and ends after a defined time cycle before it starts again to capture a subsequent frame. This time interval, i.e., the frame length, determines the maximum distance to be measured in a time-of-flight application.

A pulse is an energy signal emitted by an active depth sensing device (light-emitting diode (LED), laser, antenna, transducer, etc.) into a certain direction in 3D space. It can be emitted to several directions simultaneously forming a solid angle. The purpose of the pulse is to hit surrounding objects (such as microbubbles) and be bounced back to the sensor system as an echo.

An echo is a portion of the emitted energy pulse reflected at an object's surface and travelled back towards the sensor device. In signal processing, an echo is represented by multiple consecutive sensor amplitude samples. The time that passed from pulse emittance until an echo arrives at the sensor is known as time of arrival.

Particles refer to objects or targets of a size ranging from a maximum of 100 micrometres ($\mu$m) down to a few nanometres (nm) or less. The particle size is governed by the (medical) imaging modality and the imaging system's signal wavelength.

Microbubbles consist of a non-gaseous or gaseous core (e.g., perfluorocarbon, sulfur hexafluoride, or nitrogen) surrounded by a shell of preferably biocompatible properties (e.g., made of albumin, galactose, lipids, or polymers) and usually of a diameter between 0.5 $\mu$m and 10 $\mu$m. However, smaller and/or bigger sizes are also possible. Microbubbles are used in medical imaging as a contrast agent for ultrasound imaging. MRI contrast agents are made up of encapsulated metal compounds.

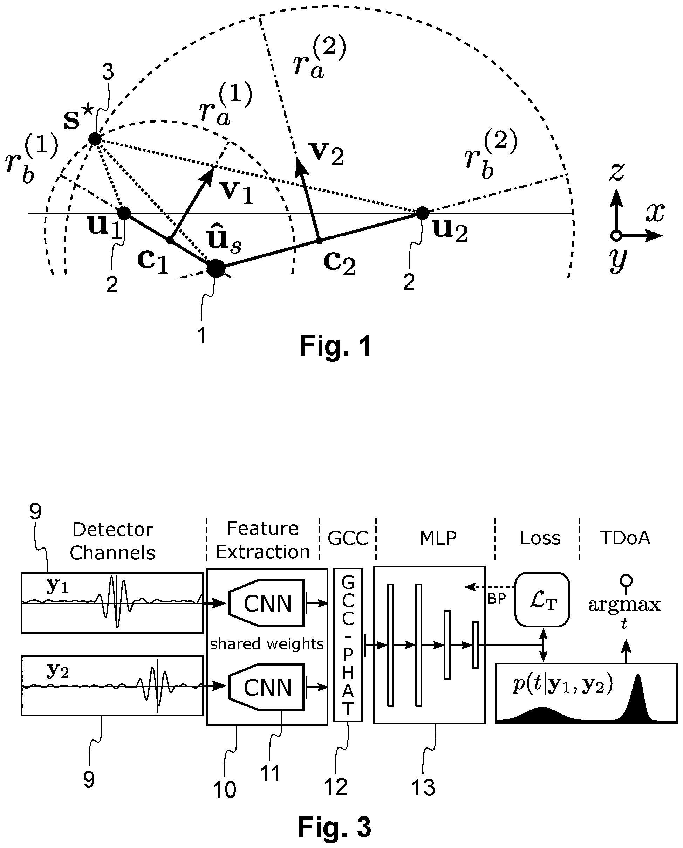

The teachings of the present invention are in the following described in more detail with reference to the figures in the context of a geometric ULM method for localising microbubbles in a human or animal body. However, the teachings of the present invention are not limited to this application. The teachings of the present invention may be applied to other medical imaging localisation methods, such as in the field of magnetic resonance imaging (MRI) or computed tomography (CT). Geometric modelling is a widely used approach for locating landmarks in space, with time-of-flight (TOF) round-trip setups being a common method. As shown in Figure 1, this setup involves a transmitter 1 and a plurality of receivers or sensors 2 (which in the present embodiment are transducers), forming a triangle between the target 3 (which in the present embodiment is a microbubble) in the present embodiment), transmitter, emitter or source 1, and receivers 2, similar to the parallax concept in visual imaging. Triangulation is then used to estimate the target's location accurately by analysing the time delay between the transmitted and received signals. However, in the single receiver case, the side lengths of the triangle are unknown, resulting in multiple candidate triangles with equal circumferences fixed at the axis connecting the receiver and the source. These candidates form an elliptical shape, as illustrated in Figure 1. By adding a second receiver, the respective ellipse intersects the first one, allowing for the resolution of the target's two-dimensional (2D) position. Figure 1 illustrates an example transducer geometry used for the ellipse intersection and localisation of a microbubble position s* from virtual source $\hat{\mathbf{u}}_s$ and receiver positions $\mathbf{u}_n$, which span ellipses rotated by $\mathbf{v}_n$ around their centres $\mathbf{c}_n$. Therefore, the accuracy of localisation depends on the ellipse model, which is parameterised by the known transducer positions and the time delays being estimated. The echo feature extraction process, which is necessary for building the ellipse intersection model accurately, is detailed later. We will also demonstrate our localisation refinement through clustering.

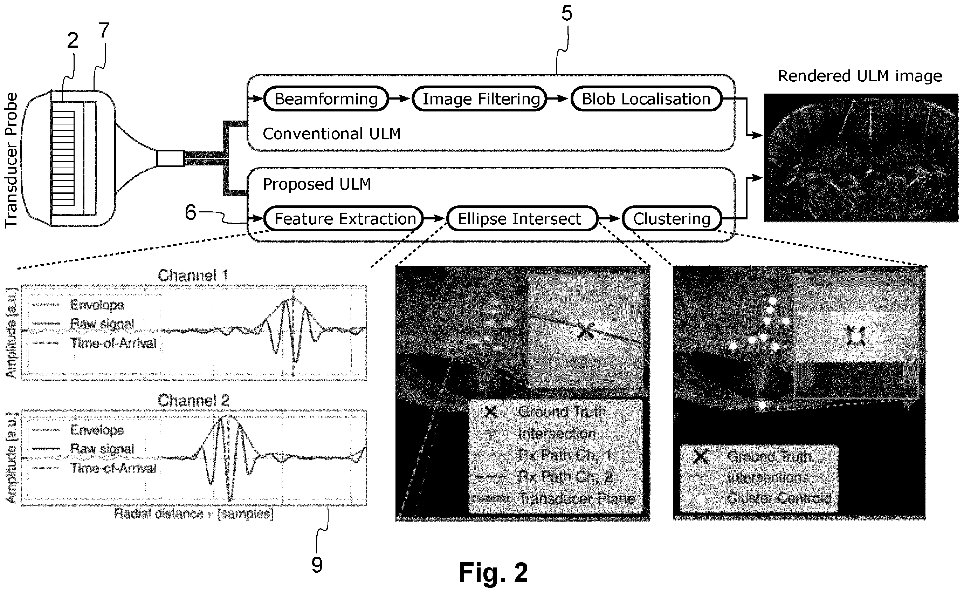

Figure 2 highlights the difference between a classical ULM pipeline 5 (top part of Figure 1) and a geometric ULM pipeline 6 according to the present invention (shown below the classical pipeline in Figure 2). A transducer probe 7 comprising a set of transducers is provided to capture echoes. The transducers may be placed arbitrarily, including an asymmetric configuration. However, a symmetric configuration of the transducers is also possible. The spatial separation between the transducers may for example be more than half a wavelength of the emitted pulse. Each transducer is associated with a respective detector channel 9. The classical ULM employs computational beamforming and image filters to localise microbubbles. According to one example, our geometric ULM comprises a cross-channel phase-consistent time of arrival detection (feature extraction and echo matching) to form ellipses that intersect at a microbubble position (ellipse intersection). As a refinement step, ellipse or ellipsoid intersections are fused via clustering as will be explained later in more detail.

In the following, we present the feature extraction stage, including a gradient-based echo detection as well as an energy-based model and a supervised deep learning network to leverage the TDOA estimation with sub-wavelength precision. The time of arrival (TOA) echo detection is first presented, followed by the energy-based and deep learning feature extraction approaches. We then discuss the limitations of the energy-based model and how the supervised network can overcome these limitations.

Initial echoes are detected by

$$t_{n,k}^\star = \{t | t \in \mathbb{R}^T \wedge \nabla_t |\mathcal{H}[y_n(t)]| > \gamma\}, \forall n \tag{1}$$

where $y_n(t)$ represents the captured amplitude data from sensor $n$. Each sample $t \in \mathbb{R}^T$ in the amplitude data qualifies as a detected TOA, denoted by $t_{n,k}^\star$, when the gradient of the Hilbert-transformed magnitude $\nabla_t |\mathcal{H}[y_n(t)]|$ surpasses a threshold value of $\gamma$.

In the following, the energy-based feature extraction model is explained in more detail. For a deterministic TOA extraction, echoes are modelled as multimodal exponentially-modified Gaussian (MEMG) distributions given by

$$f(\mathbf{m}_k; t) = \alpha_k \exp\left(-\frac{(t-\mu_k)^2}{2\sigma_k^2}\right)\left(1 + \mathrm{erf}\left(\eta_k \frac{t-\mu_k}{\sigma_k\sqrt{2}}\right)\right), \tag{2}$$

with an oscillating term that writes

$$g(\mathbf{m}_k; t) = \cos(\omega_k(t - \mu_k) + \phi_k), \tag{3}$$

where $t \in \mathbb{R}^T$ denotes the time domain with a total number of $T$ samples and $\mathbf{m}_k = [\alpha_k, \mu_k, \sigma_k, \eta_k, \omega_k, \phi_k]^\top \in \mathbb{R}^6$ contains the amplitude $\alpha_k$, mean $\mu_k$, spread $\sigma_k$, skew $\eta_k$, angular frequency $\omega_k$ and phase $\phi_k$ for each echo $k$. Note that erf(.) denotes the error function. To estimate these parameters iteratively, the cost function is given by

$$\mathcal{L}_E(\hat{\mathbf{m}}_n) = \left\| y_n(t) - \sum_{k=1}^{K} f(\mathbf{m}_k; t) g(\mathbf{m}_k; t) \right\|_2^2, \tag{4}$$

where $y_n(t)$ is the measured signal from waveform channel (i.e., detector channel) $n \in \{1, 2, ..., N\}$ and the sum over $k$ accumulates all echo components $\hat{\mathbf{m}}_n = [\mathbf{m}_1^\top, \mathbf{m}_2^\top, \mathbf{m}_k^\top, ..., \mathbf{m}_K^\top]^\top$. We get the best echo feature set $\hat{\mathbf{m}}_n^\star$ over all iterations j via

$$\hat{\mathbf{m}}_n^\star = \underset{\hat{\mathbf{m}}_n^{(j)}}{\operatorname{argmin}} \left\{ \mathcal{L}_E\left( \hat{\mathbf{m}}_n^{(j)} \right) \right\}, \tag{5}$$

for which we use the Levenberg-Marquardt solver. Model-based optimisation requires initial estimates to be nearby the solution space. For this, we assign TOAs from gradient-based analysis of the Hilbert-transformed signal in Equation 1 to set $\hat{\mathbf{m}}_n^{(1)}$.

Before geometric localisation, one should ensure that detected echo components correspond to the same microbubble. In this example, echo matching is accomplished in a heuristic brute-force fashion. Given an echo component $\mathbf{m}_{n,k}^\star$ from a reference channel index $n$, a matching echo component from an adjacent channel index $n \pm g$ with gap $g \in \mathbb{N}$ is found by $k + h$ in the neighbourhood of $h \in \{-1, 0, 1\}$. A corresponding phase-precise TOA $t_{n,k}^\star$ is obtained by $t_{n \pm g, k}^\star = \mu_{n \pm g, k+h}^\star + \phi_{n,k}^\star - \Delta$, which takes $\mu_{n,k}^\star$ and $\phi_{n,k}^\star$ from $\hat{\mathbf{m}}_n^\star$ for phase-precise alignment across transducer channels after upsampling. Here, $\Delta$ is a fixed offset to accurately capture the onset of the microbubble locations. We validate echo correspondence through a re-projection error in adjacent channels and reject those with weak alignment.

In the above-described energy-based feature extraction model, the echoes may be represented as asymmetric Gaussians. The sets of features may be extracted in a three-stage non-linear least-squares regression process. In a first stage, exponentially-modified Gaussian parameter regression of amplitude, power, TOA, and shape of the respective echo is performed. In a second stage, the amplitude, power, TOA, and shape of the respective echo are used to optimise oscillation parameters of frequency and phase of the respective echo. In a third stage, joint parameter minimisation of the amplitude, power, TOA, shape, frequency, and phase of the respective echo is performed.

In the following, we explain the deep learning model, which may be used instead of the energy-based model, for feature extraction and echo matching. Deep learning models offer several advantages over traditional energy-based approaches, such as learning representations directly from the data, which provides more robust and flexible feature extraction. Additionally, recent research has shown that deep learning models can achieve superior performance compared to traditional models.

To leverage recent advancements in convolutional neural network-based (CNN-based) TDOA detection, we employ in this example supervised feature extraction methods that build on the successful application of generalised cross-correlation (GCC) methods, such as phase transform (PHAT) in acoustic signal matching and TDOA detection using deep learning. Specifically, we follow the approach of Berg et al., "Extending GCC-PHAT using Shift Equivariant Neural Networks", in Proc. Interspeech 2022, pp. 1791-1795, who extended GCC-PHAT with shift equivariant neural networks to form a neural GCC-PHAT (NGCC-PHAT). Similarly, we use a SincNet (M. Ravanelli and Y. Bengio, "Speaker recognition from raw waveform with sincnet", in 2018 IEEE Spoken Language Technology Workshop (SLT), IEEE, 2018, pp. 1021-1028) as a backbone network, which was originally designed to recognise speakers from microphone recordings in the acoustic range of 20 Hz-20 kHz. The supervised architecture is depicted in Figure 3.

As shown in Figure 3, echoes from the detector channels 9 are fed into a feature extraction module 10 or unit comprising in this case a plurality of CNNs 11. More specifically, in this example, one CNN 11 is provided per selected echo 9. The outputs of the CNNs are fed to the generalised cross-correlation phase transform (GCC-PHAT) algorithm or module 12, which is configured to perform feature matching. The output of the GCC-PHAT is fed to another learned network 13, which in this example is a multilayer perceptron (MLP), which is configured to further process the output from the GCC-PHAT algorithm 12. This network's purpose is to learn how to effectively mitigate the effects of noise and reverberation clutter. The feature extraction process comprises thus at least one trainable neural layer for waveform data inputs with learnable weights and optional operations such as convolutional functions (e.g., for cross-correlation), normalisations, drop-out and/or attention-based filters (i.e., transformers). The functions can be swapped, i.e., interchanged to extract features, including time of arrival or time difference of arrival values for the purpose of geometric localisation in ULM.

By letting $\mathbf{y}_n = y_n(t)$, we extract features $\mathbf{x}_n$ with

$$\mathbf{x}_n = f_\theta(\mathbf{y}_n), \tag{6}$$

where $f_\theta \colon \mathbb{R}^T \mapsto \mathbb{R}^{T \times L}$ denotes the CNN 11. To obtain the TDOA between echoes, we establish the echo correlation information by means of the GCC-PHAT 12 given by

$$C[t, v] = \frac{1}{T} \sum_{u=1}^{T} \frac{X_1[u,v] X_2^*[u,v]}{|X_1[u,v] X_2^*[u,v]|} \exp\left(\frac{i 2\pi u t}{T}\right), \tag{7}$$

where $X_1$ and $X_2$ are the column-wise discrete Fourier transforms (DFTs) from feature channels $\mathbf{x}_1$ and $\mathbf{x}_2$, respectively. $X_2^*[u, v]$ denotes the complex conjugate of $X_2[u, v]$ and $C[t, v] = \boldsymbol{C} \in \mathbb{R}^{(2\tau_{max}+1) \times L}$ is the correlation matrix. These correlation values undergo further processing via

$$p(t | \mathbf{y}_1, \mathbf{y}_2) = g_\theta(C[t, l]), \tag{8}$$

where $g_\theta \colon \mathbb{R}^{(2\tau_{max}+1) \times L} \mapsto \mathbb{R}^{2\tau_{max}+1}$ is yet another artificial learned network, i.e., in this example the MLP 13, that receives the GCC-PHAT output to learn with weights $\theta$. The motivation for network learning is to eliminate the impact of noise and reverberation. The weights are trained using the cross-entropy loss function given by

$$\mathcal{L}_{\mathbf{T}}(\mathbf{y}_1, \mathbf{y}_2) = -\sum_{t=-\tau_{max}}^{\tau_{max}} \delta_\tau[t] \log p(t | \mathbf{y}_1, \mathbf{y}_2), \tag{9}$$

where $\tau_{max}$ is a maximum delay value, $\delta_\tau[t]$ is the Kronecker delta function acting as the ground-truth position, and $p(t|\mathbf{y}_1, \mathbf{y}_2)$ is the probability of delay t given $y_1$ and $y_2$. During feature inference, the TDOA is determined using

$$\tau^\star_{n \pm g, k} = \arg\max_t \{p(t | \mathbf{y}_n, \mathbf{y}_{n \pm g})\}, \tag{10}$$

where argmax (arguments of the maxima) define the points, or elements, of the domain of some function at which the function values are maximised. Thus, the overall TOA reads $t^\star_{n \pm g, k} = t^\star_{n,k} + \tau^\star_{n \pm g, k}$.

To efficiently process raw waveforms, the SincNet CNN architecture 10 in this example in the first stage of $f_\theta$ consists of parallel band-pass filters with learnable cutoff frequencies. The initial layer in this example contains 128 filters with adjustable length, followed by regular convolutions in the subsequent layers, each producing output channels of length $L$. Similarly, $g_\theta$ in this example consists of four convolutional layers with filter lengths of 11, 9, 7, and 5, all having 128 output channels, except the last layer, which generates a single output channel that models the log-probabilities for each time delay. BatchNorm and LeakyReLU activations may be applied in each layer of both networks.

The above-described supervised feature mapping for TDOA estimation can be summarised as follows. Waveform channel data $\mathbf{y}_n$ goes into the SincNet CNN 11 to generate features $\mathbf{x}_n = f_\theta(\mathbf{y}_n)$, which are cross-correlated via phase transform (by using the GCC-PHAT algorithm 12) and fed the learned network 13 $g_\theta(\mathbf{C})$ to yield probabilities $p(t|\mathbf{y}_1, \mathbf{y}_2)$. The networks $f_\theta$ and $g_\theta$ are trained simultaneously via back-propagation (BP) from a cross-entropy loss $\mathcal{L}_{\mathbf{T}}$.

We next explain the ellipse intersection determination in more detail. While ellipse intersections can be approximated iteratively, we employ Eberly's closed-form solution "Intersection of ellipses", Geometric Tools, Redmond WA 98052, Tech. Rep., 2020 owing to its fast computation property. Although one might expect that the intersection of arbitrarily placed ellipses is straightforward, it involves advanced mathematical modelling due to the degrees of freedom in the ellipse positioning. An ellipse is drawn by radii $(r_a, r_b)$ of the major and minor axes with

$$r_a = \frac{t^\star_{n,k}}{2}, \text{ and } r_b = \frac{1}{2} \sqrt{\left(t^\star_{n,k}\right)^2 - \|\hat{\mathbf{u}}_s - \mathbf{u}_n\|_2^2}, \tag{11}$$

where the virtual transmitter $\widehat{\mathbf{u}}_s \in \mathbb{R}^2$ and each receiver $\mathbf{u}_n \in \mathbb{R}^2$ with channel index $n$ represent the focal points of an ellipse, respectively. For the intersection, we begin with the ellipse standard equation. Let any point $\mathbf{s} \in \mathbb{R}^2$ located on an ellipse and displaced by its centre $\mathbf{c}_n \in \mathbb{R}^2$ be given as

$$1 = (\mathbf{s} - \mathbf{c}_n)^\top \left( \frac{\|\mathbf{v}_n\|_2^2}{r_a^2 |\mathbf{v}_n|^2} + \frac{\|\mathbf{v}_n^\perp\|_2^2}{r_b^2 |\mathbf{v}_n^\perp|^2} \right) (\mathbf{s} - \mathbf{c}_n),$$

$$= (\mathbf{s} - \mathbf{c}_n)^\top \mathbf{M}(\mathbf{s} - \mathbf{c}_n), \tag{12}$$

where M contains the ellipse equation with $\mathbf{v}_n$ and $\mathbf{v}_n^\perp$ as a pair of orthogonal ellipse direction vectors, corresponding to their radial extents $(r_0, r_1)$ as well as the squared norm $\|\cdot\|_2^2$ and vector norm $|\cdot|$. For subsequent root-finding, it is the goal to convert the standard Equation 12 to a quadratic polynomial with coefficients $b_j$ given by, $B(x,y) = b_0 + b_1 x + b_2 y + b_3 x^2 + b_4 xy = 0$, which, when written in vector-matrix form reads

$$0 = \begin{bmatrix} x & y \end{bmatrix} \begin{bmatrix} b_3 & b_4/2 \\ b_4/2 & b_5 \end{bmatrix} \begin{bmatrix} x \\ y \end{bmatrix} + \begin{bmatrix} b_1 & b_2 \end{bmatrix} \begin{bmatrix} x \\ y \end{bmatrix} + b_0 = \mathbf{s}^\top \mathbf{B}\mathbf{s} + \mathbf{b}^\top \mathbf{s} + b_0, \tag{13}$$

where **B** and **b** carry high-order polynomial coefficients $b_j$ found via matrix factorisation.

To connect Equation 13 with Equation 12, it is important to observe that $(\mathbf{s} - \mathbf{c}_n)^\top \mathbf{B}(\mathbf{s} - \mathbf{c}_n) = \mathbf{s}^\top \mathbf{B}\mathbf{s} + 2\mathbf{c}_n^\top \mathbf{B}\mathbf{s} + \mathbf{c}_n^\top \mathbf{B}\mathbf{c}_n$, which would match Equation 13 if $b_0 = \mathbf{c}_n^\top \mathbf{B}\mathbf{c}_n$ and $b = -2Bc_n$. With **B** being invertible, we rearrange the latter requirement to $\mathbf{c}_n = -\mathbf{B}^{-1}\mathbf{b}/2$ so that the above becomes

$$(\mathbf{s} - \mathbf{c}_n)^\top \mathbf{B}(\mathbf{s} - \mathbf{c}_n) = \mathbf{b}^\top \mathbf{B}^{-1}\mathbf{b}/4 - b_0,$$

and in consideration of Equation 12 consequently gives

$$\mathbf{M} = \frac{\mathbf{B}}{\mathbf{b}^\top \mathbf{B}^{-1}\mathbf{b}/4 - b_0},$$

such that polynomial coefficients $b_j$ are found via factorisation of **M**.

As an alternative to the afore-mentioned closed-form solution, iterative optimisation methods such as Gradient Descent, Gauss-Newton, Levenberg-Marquardt and other Quasi-Newton methods can be used together with cost functions to determine the ellipse intersection as disclosed for instance in European patent application No.: 22197595.6 filed on 23 September 2022.

Let two intersecting ellipses be given as quadratic equations $A(x,y)$ and $B(x,y)$ with coefficients $a_j$ and $b_j$, respectively. Their intersection is found via polynomial root-finding of the equation

$$D(x,y) = d_0 + d_1 x + d_2 y + d_3 x^2 + d_4 xy = 0,$$

where vj, $d_j = a_j - b_j$. When defining $y = w - (a_2 + a_4 x)/2$ to substitute $y$, we get $A(x, w) = w^2 + (a_0 + a_1 x + a_3 x^2) - (a_2 + a_{4x})^2/4 = 0$ which after rearranging is plugged into Equation 14 to yield an intersection point $\mathbf{s}_i^\star = [x_i, w_i]^\top$.

We describe in the following the clustering step in more detail. Microbubble reflections are dispersed across multiple waveform channels yielding groups of location candidates for the same target bubble. Localisation deviations result from TOA variations, which can occur due to atmospheric conditions, receiver clock errors, and system noise. Due to the random distribution of corresponding TOA errors, we regard these candidates as clusters. Thus, we aim to find a centroid **p**\*of each cluster using multiple bi-variate probability density functions of varying sample sizes by

$$m\left(\mathbf{p}^{(j)}\right) = \frac{\sum_{\mathbf{s}_i^\star \in \Omega^{(j)}} \exp\left(\left\|\mathbf{s}_i^\star - \mathbf{p}^{(j)}\right\|_2^2\right)\mathbf{s}_i^\star}{\sum_{\mathbf{s}_i^\star \in \Omega^{(j)}} \exp\left(\left\|\mathbf{s}_i^\star - \mathbf{p}^{(j)}\right\|_2^2\right)}. \tag{15}$$

Here, the bandwidth of the kernel is set to $\lambda/4$. The mean shift algorithm updates the estimate $\mathbf{p}^{(j)}$ by setting it to the weighted mean density on each iteration j until convergence. In this way, we obtain the position of the target bubble.

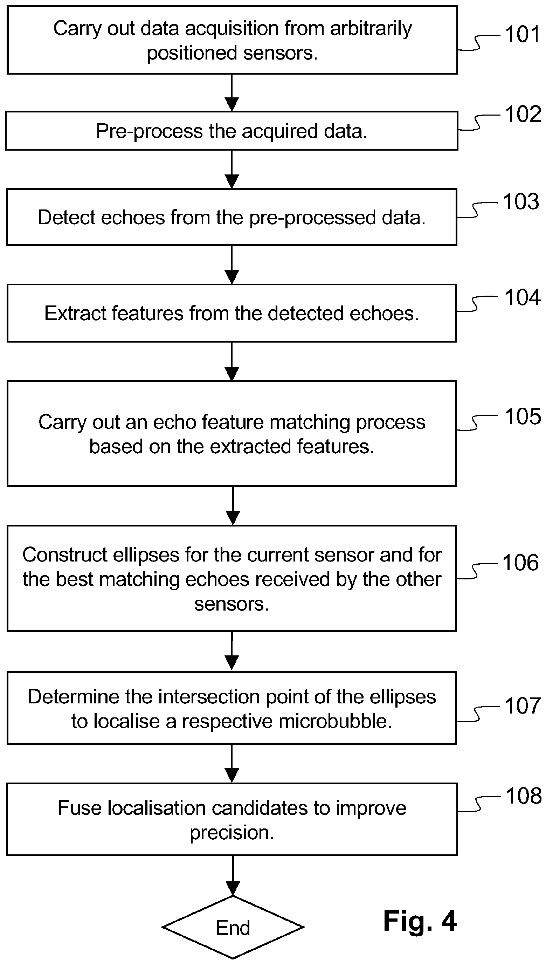

The proposed method is next illustrated with reference to the flowchart of Figure 4. In step 101, the sensors 2, which in this example are the transducers, capture data frames from the microbubbles 3 (targets). The sensors may be placed arbitrarily, and they may even move with respect to each other and the microbubbles. In other words, in this step synchronous sensor data acquisition is carried out to record target reflections. Thus, the system uses a master clock to allow the sensors to operate at identical or substantially identical rates. This means that time scales of the emitter and the sensors are aligned. As in this particular example the remaining steps are carried out by a data processing unit, step 101 also involves the sensors 2 sending the acquired data to the data processing unit, which is arranged to be in data communication with the sensors.

In step 102, the data processing unit carries out signal pre-processing. The objective of the signal pre-processing is to facilitate subsequent processing tasks such as TOA detection and echo feature extraction. The pre-processing may comprise, but is not limited to frequency domain filtering, such as band-pass filtering, compensation of the power loss, which is a result of the inverse square law, and/or Hilbert transformation of the acquired raw signal channel data.

In step 103, the data processing unit carries out echo detection across a given set of sensors 2 by using time-of-flight echo detection techniques or other suitable techniques. Echo detection in time-of-flight applications is a well-studied subject with a broad variety of solutions ranging from electronic circuitries to digital processing with algorithms employing thresholds, gradients, the Fisher matrix, dictionary-based methods and more recently convolutional neural networks.

The association of echoes that belong to a distinct target is an important step according to the present invention, which has not been satisfyingly addressed in prior work. It is important to note that matching echoes from different targets yields false positive target positions making this step an important aspect of the present invention. To address this echo correspondence problem, features are extracted in step 104 from each detected echo and compared in step 105 against echoes from other sensor channels. As explained above, feature extraction may be accomplished using various methods, such as energy-based optimisation methods (e.g., multimodal exponentially-modified Gaussians oscillators) or convolutional neural networks. Once echo features are established, the features are fed as vectors into the echo matching process, which analyses the likelihood of echoes from different sensor channels belonging together.

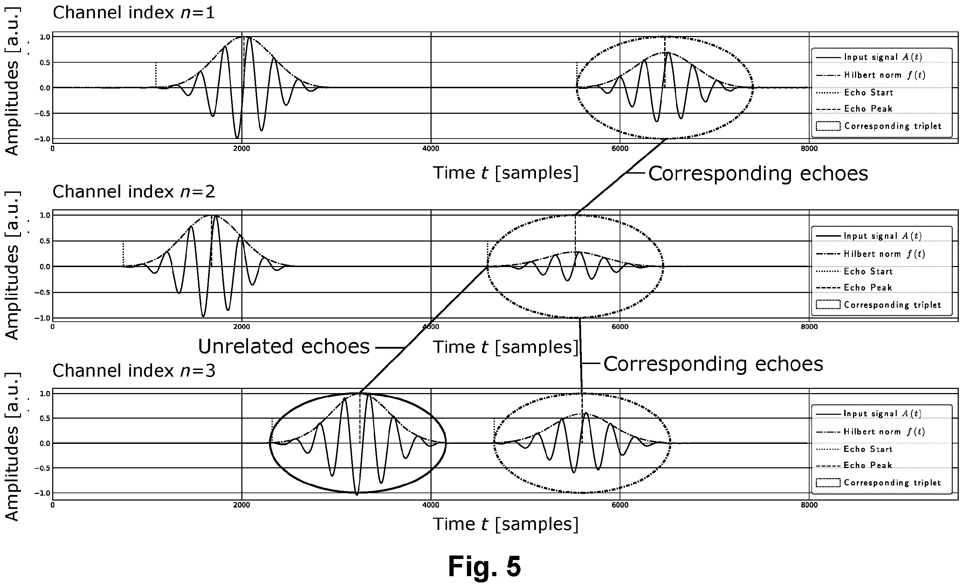

This echo correspondence likelihood or score may quantitatively be obtained by distance metrics and expressed as a so-called dissimilarity (or similarity) score. In the simplest form, such a metric is represented by the Euclidean distance. A more advanced concept as explained above comprises networks with identical neural network architectures and shared weights for each sensor channel (often referred to as Siamese networks). To train such a dissimilarity network for desired scores, a variety of losses can be employed with the contrastive loss being among the preferred ones. Figure 5 visually illustrates the echo correspondence determination.

In the case of using the neural network architectures for echo correspondence determination, then in the above example, the MLP 13 behind the cross-correlation (GCC-PHAT) yields probabilities for how similar the echoes are for each relative position shift with respect to each other. The maximum probability can be either used to determine the TDOA or (if the max is low) it is used to indicate that echoes are not similar at all. The TDOA or the probability value for a TDOA between two or more echoes can be interpreted as a correspondence likelihood or score between these echoes.

Step 105 may also involve selecting one of the echoes across different sensor channels as a reference echo. The reference echo fulfils one or more criteria for serving as a reference echo, wherein the one or more criteria are derived from echo properties including at least one or more of the following: amplitude, power, frequency, arrival time and signal shape in the form of the width, mean or/and skew in the case of Gaussian modelling. The reference echo may be the strongest echo (or one of the strongest echoes) among the different sensor channels in a given time window. Steps 104 and 105 can be considered to collectively form an echo correspondence determination process.

In step 106, ellipses or ellipsoids are mathematically constructed for the respective echoes. The ellipse generation process may be implemented so that a respective ellipse is mathematically constructed for the reference echo (in its reference sensor channel) and for each best-matching echo per sensor channel in a given time window. As an example, the length of this time window may be 500 milliseconds, 200 milliseconds, or more specifically 100 milliseconds or even 50 milliseconds and/or it may vary in length. Furthermore, in this example the time window length is decoupled from the measurement or pulse emission cycle. For instance, one may use a time window of 25 milliseconds, but only emit pulses every 50 milliseconds.

In step 107, the location of the target is determined by using an ellipse intersection model, which may be understood to be based on a zero-crossing method. A two-dimensional (2D) target position is represented by the intersection of geometric ellipses that have their foci located at sensor positions and that are spanned by detected times of arrival from corresponding sensor echo pairs. The precise intersection point retrieval can be obtained by conventional optimisation methods, such as gradient descent, conjugate gradient, Gauss-Newton, or Levenberg-Marquardt. The ellipse for each receiver is mathematically constructed within a given time window.

In step 108 which forms the optional clustering operation, the localisation candidates (i.e., a plurality of ellipse intersections) are optionally fused to obtain a precise microbubble position. The decision making by clustering methods is inherently defined. In this example, there is no training or learning involved. Most methods require some parameter settings to help in the decision-making. There is a variety of clustering methods that can be used to implement the clustering step according to the present invention. Thus, in this step, the ellipse intersections are fused based on the similarity of their properties (e.g., locations, echo amplitude). The clustering can be thought of as a classification into groups of intersection points where the classification decision is based on property similarity of the localisation candidates, such as the Cartesian coordinates (location) for instance. Other properties (e.g., echo intensity) could be used in this process additionally or instead. Optionally, the method further comprises the step of assessing the reliability of the echo correspondence, and localising the microbubbles for the data frame only if the assessed reliability is above a given threshold value for at least a given number of sensor channels.

The above-described process can be easily extended to three-dimensional (3D) localisation. In that case, instead of determining an intersection point of two ellipses, an intersection point of three ellipsoids would be determined.

The radius of each minor axis of a respective ellipsoid would be obtain as $\frac{1}{2}\sqrt{\left(t_{n,k}^{\star}\right)^2 - \|\hat{\mathbf{u}}_s - \mathbf{u}_n\|_2^2}$, while the radius of the major axis would be obtained as $\frac{t_{n,k}^{\star}}{2}$, where the source or virtual source $\hat{\mathbf{u}}_s \in \mathbb{R}^2$ and each receiver $\mathbf{u}_n \in \mathbb{R}^2$ with channel index $n$ represent the focal points of an ellipsoid. It is to be noted that it is possible to mutually swap the above-mentioned axes.

At least some of the above-described method steps may be carried out by suitable circuits or circuitry. The terms "circuits" and "circuitry" refer to physical electronic components or modules (e.g. hardware), and any software and/or firmware ("code") that may configure the hardware, be executed by the hardware, and or otherwise be associated with the hardware. The circuits may thus be operable to carry out or they comprise means for carrying out the required method steps as described above. Furthermore, at least some of the method steps can be considered as computer-implemented steps. The invention also relates to a non-transitory computer program product comprising instructions for implementing the steps or at least some of the steps of the method when loaded and run on computing means of a computing device, such as the data processing unit.

While the invention has been illustrated and described in detail in the drawings and foregoing description, such illustration and description are to be considered illustrative or exemplary and not restrictive, the invention being not limited to the disclosed embodiment. Other embodiments and variants are understood, and can be achieved by those skilled in the art when carrying out the claimed invention, based on a study of the drawings, the disclosure and the appended claims. New embodiments may be obtained by combining any of the teachings above. Furthermore, any of the above features of the present invention described in connection with the ULM method can be applied to corresponding features of the sensor system configured to implement the ULM method.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**Claims**

1. A medical imaging method for localising microbubbles and/or particles (3) by using a sensor system (7) comprising at least a source (1), and a plurality of transducers (2), the method comprising:

   • receiving (101) a set of data frames in response to a set of pulses being reflected from a set of microbubbles and/or particles (3) to be localised, a respective data frame being associated with a sensor channel (9);
   • pre-processing (102) the data frames to facilitate subsequent processing of the data frames;
   • detecting (103) echoes and times of arrival of the echoes from the pre-processed data frames;
   • extracting (104) sets of features from the detected echoes;
   • comparing (105) the extracted set of features in a given time window of a respective sensor channel to corresponding sets of features from other sensor channels to obtain a respective echo correspondence score between the extracted set of features and a respective set of features of a respective echo of a respective other sensor channel, the respective echo correspondence score indicating directly or indirectly the likelihood that the respective echoes are reflected from the same microbubble or particle;

• mathematically constructing (106) a respective ellipse or ellipsoid for the detected echo of the respective sensor channel and for the respective echo for which the echo correspondence score indicates the best echo correspondence in at least the respective one other sensor channel in the given time window to obtain a set of ellipses or ellipsoids; and

• determining (107) an intersection point of the ellipses or ellipsoids in the set of ellipses or ellipsoids to localise a respective microbubble or particle.

2. A method according to claim 1, wherein a first radius of the respective ellipse or ellipsoid is obtained from a respective time of arrival of the respective echo for which the respective ellipsoid is constructed, and a second radius of the respective ellipse or ellipsoid is obtained from the respective time of arrival of the respective echo for which the respective ellipsoid is constructed, and the location of the source (1) and the respective transducer (2).

3. A method according to claim 2, wherein a first axis of the respective ellipse or ellipsoid equals $\dfrac{t_{n,k}^{\star}}{2}$, and at least a second axis equals $\dfrac{1}{2}\sqrt{\left(t_{n,k}^{\star}\right)^{2}-\|\widehat{\mathbf{u}}_{s}-\mathbf{u}_{n}\|_{2}^{2}}$, where $\widehat{\mathbf{u}}_{s}\in\mathbb{R}^{2}$ or $\mathbb{R}^{3}$ is the source position at a first focal point of the respective ellipse or ellipsoid, $\mathbf{u}_{n}\in\mathbb{R}^{2}$ or $\mathbb{R}^{3}$ is the respective transducer position at a second focal point of the respective ellipse or ellipsoid, and $t_{n,k}^{\star}$ denotes the time of arrival of the respective echo.

4. A method according to any one of the preceding claims, wherein the features in the extracted sets of features comprise any of the following features relating to the respective echo: amplitude, power, time difference of arrival between two or more echoes, probabilities of time difference of arrival values between two or more echoes, time of arrival and shape as described by the mean, and/or spread, and/or skew when the echo is modelled as a Gaussian distribution, as well as frequency and/or phase for oscillation modelling of the echo, and/or wherein the set of ellipses comprises at least two ellipses, or wherein the set of ellipsoids comprises at least three ellipsoids.

5. A method according to any one of the preceding claims, wherein the respective echo correspondence score is obtained as a distance metric computed feature-wise between the extracted set of features of the respective sensor channel and the respective set of features of the respective echo of the respective other sensor channel, or wherein the respective echo correspondence score comprises a probability value for a time difference of arrival value between two or more echoes.

6. A method according to any one of the preceding claims, wherein the sets of features are extracted by using an energy-optimisation-based method and/or by using one or more convolutional neural networks (11).

7. A method according to claim 6, wherein the energy-optimisation-based method involves using a multimodal exponentially-modified Gaussian model, and wherein the multimodal exponentially-modified Gaussian model comprises one or more oscillation terms.

8. A method according to claim 7, wherein the echoes are represented as asymmetric Gaussians, and wherein the sets of features are extracted in a three-stage non-linear least-squares regression process, wherein in a first stage, exponentially-modified Gaussian parameter regression of amplitude, power, time of arrival, and shape of the respective echo is performed, in a second stage, the amplitude, power, time of arrival, and shape of the respective echo are used to optimise oscillation parameters of frequency and phase of the respective echo, and in a third stage, joint parameter minimisation of the amplitude, power, time of arrival, shape, frequency, and phase of the respective echo is performed.

9. A method according to any one of the preceding claims, wherein the sets of features are extracted by using one or more convolutional neural networks (11), and wherein the extracted features are compared by using a cross-correlation algorithm (12).

10. A method according to claim 9, wherein an output of the cross-correlation algorithm (12) is processed by a learned network (13) generating probabilities of time difference of arrival values between two or more echoes, wherein the learned network (13) is a multi-layer perceptron network, and wherein the learned network (13) and/or the one or more convolutional neural networks (11) is/are trained with one or more loss terms through a backpropagation arrangement.

11. A method according to any one of the preceding claims, wherein the method further comprises fusing (108) a plurality of ellipse or ellipsoid intersections to more accurately locate the microbubbles and/or particles.

12. A method according to any one of the preceding claims, wherein the detected echo of the respective sensor channel is a reference echo fulfilling one or more criteria for serving as a reference echo, wherein the one or more criteria are derived from echo properties including at least one or more of the following: amplitude, power, time of arrival, frequency, phase, mean, spread, and skew for the asymmetric Gaussian modelling of the echo, and/or the method further comprises assessing the reliability of the echo correspondence, and localising the respective microbubble or particle for the respective data frame only if the assessed reliability is above a given threshold value for at least a given number of sensor channels.

13. A method according to any one of the preceding claims, wherein the pre-processing of the data frames comprises at least one of the following operations: spectral filtering of the data frames, power loss compensation of the data frames, and Hilbert transformation of the data frames.

14. A method according to any one of the preceding claims, wherein the method comprises placing the transducers (2) symmetrically with respect to each other or placing the transducers (2) arbitrarily, and determining relative and/or absolute positions of the transducers (2), and/or wherein at least two of the transducers (2) operate in mutually different frequency bands.

15. A sensor system (7) for performing a medical imaging method for localising microbubbles and/or particles (3), the sensor system (7) comprising at least a source (1) and a plurality of transducers (2), the sensor system comprising means for:

   • receiving a set of data frames in response to a set of pulses being reflected from a set of microbubbles and/or particles (3) to be localised, a respective data frame being associated with a sensor channel (9);
   • pre-processing the data frames to facilitate subsequent processing of the data frames;
   • detecting echoes and times of arrival of the echoes from the pre-processed data frames;
   • extracting sets of features from the detected echoes;
   • comparing the extracted set of features in a given time window of a respective sensor channel to corresponding sets of features from other sensor channels to obtain a respective echo correspondence score between the extracted set of features and a respective set of features of a respective echo of a respective other sensor channel, the respective echo correspondence score indicating directly or indirectly the likelihood that the respective echoes are reflected from the same microbubble or particle;
   • mathematically constructing a respective ellipse or ellipsoid for the detected echo of the respective sensor channel and for the respective echo for which the echo correspondence score indicates the best echo correspondence in at least the respective one other sensor channel in the given time window to obtain a set of ellipses or ellipsoids; and
   • determining an intersection point of the ellipses or ellipsoids in the set of ellipses or ellipsoids to localise a respective microbubble or particle.

**Fig. 1**

**Fig. 3**

Fig. 2

```
┌─────────────────────────────────────┐
│   Carry out data acquisition from    │      ╭─ 101
│   arbitrarily positioned sensors.    │
└─────────────────────────────────────┘
                   │
                   ▼
┌─────────────────────────────────────┐
│      Pre-process the acquired data.  │      ╭─ 102
└─────────────────────────────────────┘
                   │
                   ▼
┌─────────────────────────────────────┐
│  Detect echoes from the pre-         │      ╭─ 103
│  processed data.                     │
└─────────────────────────────────────┘
                   │
                   ▼
┌─────────────────────────────────────┐
│  Extract features from the detected  │      ╭─ 104
│  echoes.                             │
└─────────────────────────────────────┘
                   │
                   ▼
┌─────────────────────────────────────┐
│  Carry out an echo feature matching  │      ╭─ 105
│  process based on the extracted      │
│  features.                           │
└─────────────────────────────────────┘
                   │
                   ▼
┌─────────────────────────────────────┐
│  Construct ellipses for the current  │      ╭─ 106
│  sensor and for the best matching    │
│  echoes received by the other        │
│  sensors.                            │
└─────────────────────────────────────┘
                   │
                   ▼
┌─────────────────────────────────────┐
│  Determine the intersection point    │      ╭─ 107
│  of the ellipses to localise a       │
│  respective microbubble.             │
└─────────────────────────────────────┘
                   │
                   ▼
┌─────────────────────────────────────┐
│  Fuse localisation candidates to     │      ╭─ 108
│  improve precision.                  │
└─────────────────────────────────────┘
                   │
                   ▼
              ◇ End ◇                        **Fig. 4**
```

Fig. 5

EP 4 467 081 A1

Europäisches
Patentamt

European
Patent Office

Office européen
des brevets

# EUROPEAN SEARCH REPORT

Application Number

EP 23 17 4686

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | HAHNE CHRISTOPHER ET AL: "Geometric Ultrasound Localization Microscopy", 1 October 2023 (2023-10-01), MEDICAL IMAGE COMPUTING AND COMPUTER ASSISTED INTERVENTION — MICCAI 2023; [LECTURE NOTES IN COMPUTER SCIENCE; LECT.NOTES COMPUTER], SPRINGER NATURE SWITZERLAND, CHAM, PAGE(S) 217 — 227, XP047671793, ISSN: 0302-9743 ISBN: 978-3-031-43998-8 [retrieved on 2023-10-01] * abstract * | 1-15 | INV. A61B8/00 A61B8/08 |
| A,P | CHRISTOPHER HAHNE ET AL: "Geometric Ultrasound Localization Microscopy", ARXIV.ORG, CORNELL UNIVERSITY LIBRARY, 201 OLIN LIBRARY CORNELL UNIVERSITY ITHACA, NY 14853, 18 July 2023 (2023-07-18), XP091565331, * abstract * | 1-15 | |
| A | US 2022/000454 A1 (DUPLAT BERTRAND [FR] ET AL) 6 January 2022 (2022-01-06) * paragraphs [0001], [0005] – [0008], [0019] – [0035], [0066] – [0070] * | 1-15 | TECHNICAL FIELDS SEARCHED (IPC) A61B |
| A | US 2020/187910 A1 (PINTON GIANMARCO FRANCESCO [US] ET AL) 18 June 2020 (2020-06-18) * paragraphs [0005] – [0027], [0086]; claims; figures * | 1-15 | |

-/--

The present search report has been drawn up for all claims

| Place of search | Date of completion of the search | Examiner |
|---|---|---|
| Munich | 17 October 2023 | Mundakapadam, S |

EPO FORM 1503 03.82 (P04C01)

page 1 of 2

## EUROPEAN SEARCH REPORT

Application Number

EP 23 17 4686

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VAN SLOUN RUUD J G ET AL: "Super-Resolution Ultrasound Localization Microscopy Through Deep Learning", IEEE TRANSACTIONS ON MEDICAL IMAGING, IEEE, USA, vol. 40, no. 3, 16 November 2020 (2020-11-16), pages 829-839, XP011840877, ISSN: 0278-0062, DOI: 10.1109/TMI.2020.3037790 [retrieved on 2021-03-02] * abstract * | 1-15 | |
| A | BOLOGNI GIOVANNI ET AL: "Acoustic Reflectors Localization from Stereo Recordings Using Neural Networks", ICASSP 2021 - 2021 IEEE INTERNATIONAL CONFERENCE ON ACOUSTICS, SPEECH AND SIGNAL PROCESSING (ICASSP), IEEE, 6 June 2021 (2021-06-06), pages 1-5, XP033954603, DOI: 10.1109/ICASSP39728.2021.9414473 [retrieved on 2021-04-22] * abstract * | 1-15 | **TECHNICAL FIELDS SEARCHED (IPC)** |

The present search report has been drawn up for all claims

| Place of search | Date of completion of the search | Examiner |
|---|---|---|
| Munich | 17 October 2023 | Mundakapadam, S |

EPO FORM 1503 03.82 (P04C01)

## ANNEX TO THE EUROPEAN SEARCH REPORT
## ON EUROPEAN PATENT APPLICATION NO.

EP 23 17 4686

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2023

| Patent document cited in search report | Publication date | Patent family member(s) | | Publication date |
|---|---|---|---|---|
| US 2022000454 A1 | 06-01-2022 | EP | 3880081 A1 | 22-09-2021 |
| | | US | 2022000454 A1 | 06-01-2022 |
| | | WO | 2020135945 A1 | 02-07-2020 |
| US 2020187910 A1 | 18-06-2020 | US | 2020187910 A1 | 18-06-2020 |
| | | WO | 2018208942 A1 | 15-11-2018 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 22197595

**Non-patent literature cited in the description**

- **ERRICO et al.** Ultrafast ultrasound localization microscopy for deep super-resolution vascular imaging. *Nature*, 2015, vol. 527 (7579), 499-502
- **BERG et al.** Extending GCC-PHAT using Shift Equivariant Neural Networks. *Proc. Interspeech*, 2022, 1791-1795
- Speaker recognition from raw waveform with sincnet. **M. RAVANELLI** ; **Y. BENGIO**. 2018 IEEE Spoken Language Technology Workshop (SLT). IEEE, 2018, 1021-1028
- Intersection of ellipses. *Tech. Rep.*, 2020